# FASCICULE DE BREVET EUROPEEN

(11) **EP 2 806 974 B1**
(45) Date de publication et mention de la délivrance du brevet: **29.05.2019**
(21) Numéro de dépôt: 13704207.3
(22) Date de dépôt: 25.01.2013
(51) Int. Cl.: B02C 19/00

(54) **AUTOMATE ET PROCÉDÉ DE TRAITEMENT DE DÉCHETS**
VERFAHREN UND VORRICHTUNG ZUR ABFALLBEHANDLUNG
PROCESS AND WASTE TREATMENT DEVICE

(30) Priorité: 27.01.2012 FR 1250806
(43) Date de publication de la demande: 03.12.2014
(73) Titulaire: Bertin Technologies, 78180 Montigny-le-Bretonneux (FR)
(72) Inventeur: ROCH, Jean, F-13109 Simiane Collongue (FR); LORECKI, Boguslaw, F-13850 Greasque (FR); BESNARD, Jacques, F-78180 Montigny Le Bretonneux (FR)
(74) Mandataire: Decobert, Jean-Pascal
(86) Numéro de dépôt international: PCT/FR2013/050158
(87) Numéro de publication internationale: WO 2013/110900

(56) Documents cités:
- EP-B1- 1 635 883
- FR-A1- 2 757 063
- US-A- 5 270 000
- US-A- 5 792 421

## Description

La présente invention se rapporte à un automate de traitement de déchets, en particulier de banalisation de déchets d'activités de soins à risques infectieux (appelés DASRI), ainsi qu'à un procédé de traitement de déchets utilisant cet automate.

Les déchets du type DASRI sont générés par les centres de soins tels que les hôpitaux, les cliniques, etc. Ils comprennent notamment les matériels et matériaux de soins piquants, coupants et/ou tranchants, les produits sanguins à usage thérapeutique, les déchets anatomiques humains, les déchets issus des activités d'enseignement, de recherche et de production industrielle dans les domaines de la médecine humaine et vétérinaire, etc. (Art. R1335-1 du Code de la Santé Publique de la République Française).

Ces déchets présentent un risque infectieux du fait qu'ils peuvent contenir des micro-organismes. Ils suivent donc des filières d'élimination spécifiques, étroitement encadrées par la législation française.

Les centres de soins équipés d'un système de banalisation des déchets du type DASRI peuvent traiter eux-mêmes les déchets qu'ils produisent, alors que les déchets de type DASRI qui sont produits par les centres de soins non équipés d'un tel système doivent être récoltés par des entreprises spécialisées et traités dans des centres d'incinération et de banalisation dédiés et agréés pour le traitement de ce type de déchets.

Un système de banalisation de déchets du type DASRI doit être capable d'une part de transformer les déchets pour les rendre méconnaissables (en particulier pour empêcher de reconnaître leur provenance ou origine) et d'autre part de les désinfecter, c'est-à-dire de réduire leur population bactérienne et virale d'un facteur au moins égal à 5 log10.

De nombreux systèmes de banalisation existent, ces systèmes intégrant une fonction de broyage associée à une fonction de chauffage des déchets, en vue de leur. Un dispositif de stérilisation de déchets médicaux infectieux comportant des moyens de broyage des déchets ainsi que des moyens de chauffage par micro-ondes situés en aval des moyens de broyage est par exemple décrit dans le document FR 2 757 063 A1.

La présente invention a notamment pour but de fournir une autre technologie capable de traiter des déchets, et en particulier de banaliser des déchets du type DASRI, d'une manière automatisée, efficace et économique.

Elle propose à cet effet un automate de traitement de déchets, en particulier de banalisation de déchets d'activités de soins à risques infectieux, comprenant une cuve montée sur un châssis et destinée à être alimentée en déchets, des moyens de broyage des déchets, par exemple du type à lame rotative, montés au fond de la cuve, des moyens de chauffage des déchets en vue de leur désinfection, et des moyens informatisés de commande des moyens de broyage et de chauffage, caractérisé en ce que les moyens de chauffage comprennent au moins un générateur de micro-ondes dont la sortie est reliée à une extrémité d'un guide d'onde dont l'autre extrémité débouche dans la cuve, le générateur étant porté par des moyens de support indépendants du châssis ou montés sur le châssis par des moyens absorbant les vibrations de la cuve lors du broyage des déchets.

Selon l'invention, la désinfection des déchets est obtenue par l'émission de micro-ondes dans la cuve, ces micro-ondes étant destinées à chauffer les déchets jusqu'à une température qui peut être maintenue pendant une durée suffisante pour réduire la population bactérienne et virale des déchets d'un facteur minimum de 5 log10, comme l'exigent les normes applicables dans certains pays. La granulométrie des déchets est en outre réduite par broyage. Le broyage, le chauffage et la désinfection des déchets sont réalisés dans la même cuve et permettent de banaliser les déchets, c'est-à-dire de les rendre non-identifiables, les déchets banalisés pouvant éventuellement être mélangés à des ordures ménagères classiques.

En fonctionnement, le broyage des déchets dans la cuve génère des vibrations importantes, qui sont susceptibles de perturber le fonctionnement du générateur de micro-ondes et de le dégrader. Pour limiter voire empêcher ce phénomène, le générateur de micro-ondes est relié à la cuve par un guide d'onde et est supporté par des moyens indépendants du châssis de support de la cuve ou montés sur le châssis par des moyens d'absorption des vibrations de la cuve lors du broyage. Les vibrations auxquelles la cuve est soumise en fonctionnement ne sont ainsi pas ou peu transmises au générateur, ce qui garantit son bon fonctionnement et augmente sa durée de vie.

Avantageusement, le guide d'onde peut comprendre au moins une partie souple ou flexible, de façon à limiter ou empêcher la transmission de vibrations de la cuve au générateur via le guide d'onde. Cette partie souple ou flexible peut être située à l'extrémité du guide d'onde située du côté de la cuve. Un guide d'onde souple peut être un guide d'onde métallique à parois ondulées, ce guide d'onde ayant une capacité de flexion et de torsion tout en maintenant une qualité et une stabilité de transmission électromagnétique. Ce type de guide remplace en lieu et place un guide rectangulaire standard pour isoler les vibrations et éliminer des difficultés causées par l'installation telles que le positionnement et l'alignement des guides de cette installation. La surface extérieure du guide souple peut être ou non revêtue de différents matériaux de protection pour convenir à la plupart des exigences en termes de vibrations.

Selon une autre caractéristique de l'invention, le guide d'onde débouche à son extrémité opposée au générateur sur un hublot de la cuve, à travers lequel sont transmises les micro-ondes. Ce hublot peut être réalisé dans un matériau polymère diélectrique approprié, tel que par exemple en Téflon®, PP, PEEK, etc.

La cuve de l'automate peut avoir une forme générale cylindrique et comprendre une paroi cylindrique dont l'axe de révolution s'étend sensiblement verticalement. Les moyens de broyage sont montés au fond de la cuve et sont donc situés dans une demie partie inférieure de la cuve. Le hublot d'émission des micro-ondes est de préférence situé dans la demie partie supérieure de la cuve, de façon à ce que les micro-ondes soient émises au niveau d'une couche supérieure de déchets dans la cuve.

Dans le cas où les moyens de broyage comprennent une lame rotative, pour garantir une homogénéité de température des déchets à l'intérieur de la cuve, la lame rotative effectue de préférence une rotation à faible vitesse (de quelques tours par minute) afin de brasser les déchets et permettre une exposition aux micro-ondes quasi-uniforme de tous les déchets.

La cuve peut avoir un volume interne variant de quelques dizaines de litres à plusieurs centaines de litres. Dans un cas particulier de réalisation de l'invention, la cuve a un volume interne de 440L environ.

Le générateur de micro-ondes est de préférence un magnétron qui fonctionne à une fréquence comprise entre 1 et 3 GHz, et par exemple de 2,45 GHz environ. Si nécessaire, l'automate peut comprendre deux magnétrons qui sont reliés chacun par un guide d'onde à la cuve.

Le guide d'onde peut comprendre un isolateur destiné à autoriser le passage des ondes émises par le générateur vers la cuve et à empêcher le retour des ondes réfléchies depuis la cuve vers le générateur. En effet, certains déchets, en particulier ceux métalliques, peuvent réfléchir les ondes qui ne doivent pas revenir vers le magnétron, car cela pourrait l'endommager.

Dans un mode particulier de réalisation de l'invention, le guide d'onde comprend un circulateur à trois voies équipé d'un isolateur à charge absorbante, le circulateur comportant une première voie reliée par une portion de guide d'onde à la sortie du générateur, une seconde voie reliée par une portion de guide d'onde à la cuve, et une troisième voie reliée à la charge absorbante, l'isolateur comportant un noyau en ferrite à aimentation permanente disposé au centre du circulateur et destiné à forcer les ondes incidentes provenant de la première voie à passer dans la seconde voie et les ondes réfléchies de la seconde voie à passer dans la troisième voie.

Alternativement ou en caractéristique additionnelle, le guide d'onde peut comprendre un adaptateur d'impédance, par exemple du type à piston(s) ou tige(s) plongeant(s).

Avantageusement, la cuve comprend des moyens empêchant ou limitant les fuites d'ondes tels que des pièges quart d'onde, des pièges comportant des matériaux absorbants, des joints formés de tresses métalliques, et/ou des jeux résiduels de montage entre certaines pièces de la cuve pour éviter la formation d'arcs électriques.

L'automate selon l'invention peut en outre comprendre un ou plusieurs des équipements suivants :
- au moins un capteur de la température et/ou de l'humidité à l'intérieur de la cuve ;
- un système de refroidissement du générateur par circulation d'eau autour, à proximité ou à l'intérieur de celui-ci ;
- un ou plusieurs systèmes de filtration de gaz permettant de traiter les vapeurs et les effluents olfactifs générés lors du traitement des déchets, ce système comportant une entrée reliée par un conduit à un orifice d'évacuation de gaz de la cuve, dont l'ouverture peut être commandée par une vanne ;
- des moyens de pesée des déchets avant leur introduction dans la cuve et/ou après leur traitement ;
- une plate-forme surélevée de contrôle qui est accessible par un opérateur, et qui est située à côté du châssis de support de la cuve et des moyens de commande ;
- des moyens de récupération et de stockage des déchets après traitement, ces moyens de récupération étant logés sous la cuve qui comporte au niveau ou au voisinage de son fond au moins une trappe d'évacuation des déchets et de vidange de la cuve ;
- au moins un organe en saillie sur une paroi cylindrique interne de la cuve, cet organe formant un obstacle pour les déchets lors du broyage ; ces organes sont de préférence situés au dessus des moyens de broyage ; dans le cas où ces moyens de broyage comprennent une lame rotative, ils permettent d'éviter un phénomène de centrifugation des déchets lors de la phase de broyage par rotation de la lame à grande vitesse (quelques centaines à quelques milliers de tours par minute) ; et/ou
- un couvercle de fermeture étanche de la cuve, ce couvercle étant monté pivotant à l'extrémité supérieure de la cuve entre une position d'ouverture et une position de fermeture de la cuve, et des moyens de verrouillage du couvercle en position de fermeture, qui sont commandés par les moyens de commande.

L'invention concerne également un procédé automatisé de traitement de déchets, au moyen d'un automate tel que décrit ci-dessus, cet automate comportant des moyens de broyage des déchets du type à lame tournante, caractérisé en ce qu'il comprend les étapes consistant à :
a) introduire les déchets dans la cuve ;
b) broyer les déchets par rotation des lames à une vitesse V1 donnée, le broyage produisant de la chaleur chauffant les déchets ;
c) émettre des micro-ondes dans la cuve en brassant les déchets par rotation de la lame à une vitesse V3 inférieure à V1, pour chauffer les déchets et les maintenir à une température de désinfection pendant une durée déterminée ; et
d) évacuer les déchets de la cuve.

Le procédé selon l'invention peut comprendre une ou plusieurs des étapes suivantes :
- avant l'étape a) et/ou après l'étape d), une étape de pesée des déchets ;
- après l'étape a), une étape de fermeture et de verrouillage du couvercle de la cuve ;
- avant l'étape b), une étape de pré-broyage des déchets par rotation de la lame à une vitesse V4 inférieure à V1 ;
- pendant l'étape c), une étape de mesure de la température à l'intérieur de la cuve ;
- après l'étape d), une étape d'enregistrement des données du traitement par les moyens de commande ; et/ou
- une étape de test de désinfection comportant, avant l'étape c), une sous-étape d'introduction d'échantillons témoins de désinfection dans la cuve, et, après l'étape d), une sous-étape de récupération des échantillons précités en vue de leur analyse.

L'automate et le procédé selon l'invention permettent de réduire de 80% le volume des déchets. Ils peuvent en outre permettre de réduire de 20% la masse des déchets en vaporisant et en éliminant l'eau qu'ils contiennent. L'invention peut donc réduire de manière significative à la fois la masse et le volume des déchets à traiter.

L'invention sera mieux comprise et d'autres détails, avantages et caractéristiques de l'invention apparaîtront plus clairement à la lecture de la description suivante faite à titre d'exemple non limitatif et en référence aux dessins annexés dans lesquels :
- la figure 1 est une vue schématique en perspective de l'automate de traitement de déchets selon l'invention ;
- les figures 2 et 3 sont des vues schématiques partielles de l'automate de la figure 1, vu de dessus et en coupe axiale, respectivement ;
- la figure 4 est une autre vue schématique en perspective de l'automate de la figure 1 ;
- la figure 5 est une autre vue schématique en perspective de la cuve de l'automate de la figure 1 ;
- la figure 6 est une vue schématique en coupe axiale de la cuve de la figure 5 ;
- la figure 7 est une vue schématique des moyens de broyage de l'automate de la figure 1 ;
- les figures 8 et 9 sont des vues très schématiques du guide d'onde de liaison du générateur de micro-ondes à la cuve de l'automate de la figure 1, ce guide d'onde comprenant un isolateur à charge absorbante et un adaptateur d'impédance ;
- la figure 10 est une vue schématique en perspective de l'adaptateur d'impédance du guide d'onde des figures 8 et 9 ;
- la figure 11 est un organigramme présentant des étapes du procédé selon l'invention de traitement de déchets ;
- la figure 12 est un graphe représentant l'évolution au cours du temps de la vitesse de rotation des moyens de broyage de l'automate et de la température dans la cuve ; et
- les figures 13 et 14 sont des vues schématiques en coupe axiale de la cuve de l'automate, et représentent des sous-étapes d'un test de désinfection du procédé selon l'invention.

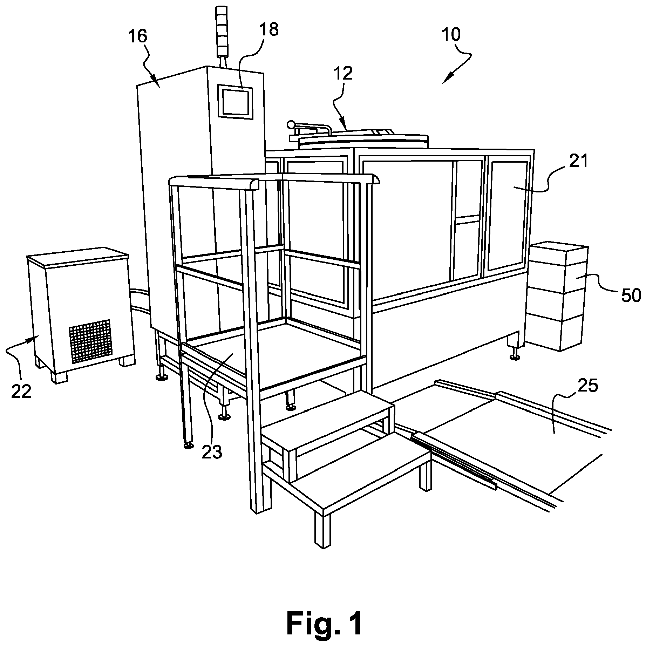

On se réfère d'abord aux figures 1 à 4 qui représentent un exemple de réalisation de l'automate 10 de traitement de déchets selon l'invention, cet automate étant particulièrement mais non exclusivement destiné à la banalisation de déchets d'activités de soins à risques infectieux (« DASRI »).

Typiquement, les déchets du type DASRI sont stockés dans des conditionnements tels que des sacs de collecte (en polyéthylène), des récipients ou boîtes de volume compris entre 0,3 et 10L (par exemple des boîtes à aiguilles en polypropylène), et des cartons dont le volume est compris entre 12 et 50L.

Dans l'exemple représenté, l'automate 10 comprend essentiellement trois éléments :
- une cuve 12 de traitement des déchets, dans laquelle les déchets sont destinés à être broyés et chauffés en vue de leur traitement, ces déchets étant introduits dans la cuve avec leurs conditionnements qui sont également destinés à être broyés ;
- un générateur 14 de micro-ondes pour l'alimentation de la cuve 12 en micro-ondes destinées à chauffer les déchets en vue de leur désinfection ; et
- des moyens informatisés 16 de commande reliés à la cuve 12 et au générateur 14 pour leur commande ainsi que pour la saisie et l'enregistrement de données et la gestion du procédé de traitement des déchets.

Les moyens de commande 16 comprennent une armoire électrique qui comporte notamment un écran 18 d'affichage d'informations et de contrôle du procédé de traitement, ainsi que des moyens de saisie d'informations. De préférence, l'écran 18 est du type tactile et un opérateur peut saisir des informations et effectuer des requêtes directement par l'intermédiaire de cet écran 18. L'armoire électrique des moyens de commande 16 est directement posée au sol.

Le générateur 14 de micro-ondes est un magnétron qui fonctionne à une fréquence comprise entre 1 et 3 GHz. Dans un mode particulier de réalisation de l'invention, le magnétron présente les caractéristiques suivantes : fréquence de 2450MHz, puissance de 3kW en multi-mode.

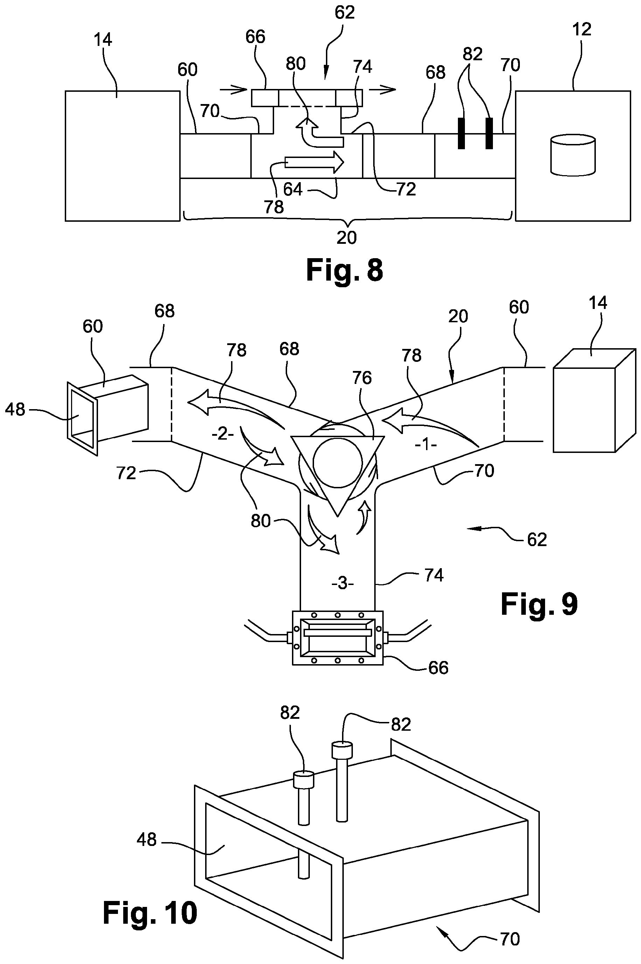

Le magnétron 14 est relié à la cuve 12 par un guide d'onde 20 qui sera décrit plus en détail dans ce qui suit en référence aux figures 8 à 10. Le guide d'onde assure un couplage de puissance entre le magnétron 14 et la cuve 12. Dans l'exemple représenté, le guide d'onde 20 comprend une partie d'extrémité reliée à la cuve 12 qui est plus souple ou plus flexible que le reste du guide d'onde. Cette partie de plus grande souplesse ou flexibilité peut être formée par une portion de guide d'onde à parois métalliques ondulées. La surface extérieure de cette portion peut être ou non revêtue de différents matériaux de protection pour convenir à la plupart des exigences en termes de vibrations.

En fonctionnement, le magnétron 14 peut être alimenté en eau en vue de son refroidissement. Dans l'exemple représenté, l'automate 10 comprend un refroidisseur 22 à eau (figure 1) dont une entrée est reliée à une extrémité d'un circuit d'eau du magnétron et une sortie est reliée à une autre extrémité de ce circuit d'eau. Le magnétron 14 peut être alimenté en eau à une pression de 3 bars et à un débit de 3L/min environ. Le refroidisseur 22 utilisé est par exemple celui commercialisé sous la marque Donaldson ultrafilter, modèle Ultracool mini UC20, qui a une capacité de refroidissement de 1,96kW et un débit d'eau maximal de 337L/h.

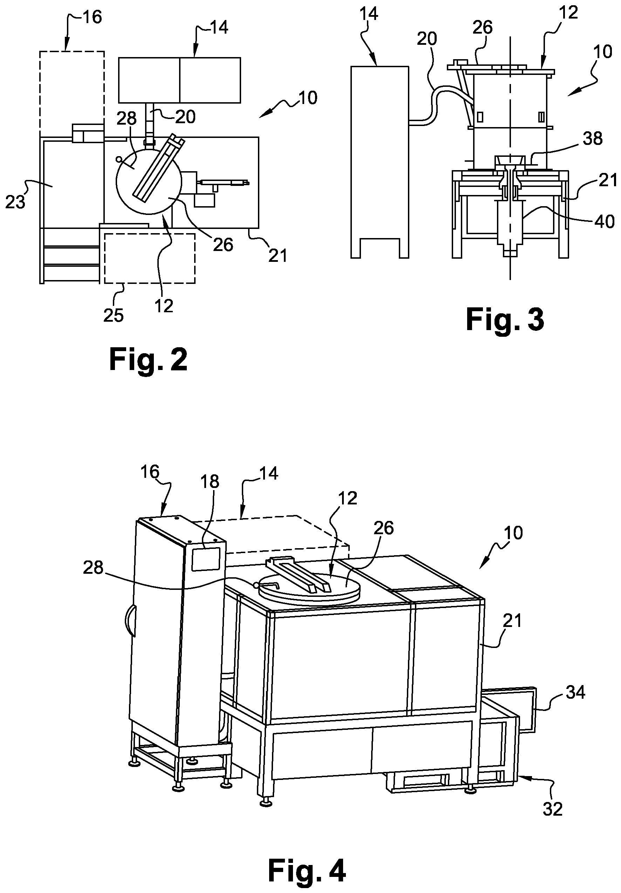

L'automate 10 comprend un châssis 21 de support de la cuve 10, ce châssis étant directement posé au sol et indépendant des moyens de support du générateur 14 pour éviter la transmission de vibrations par le châssis en fonctionnement, comme expliqué dans ce qui précède. Dans l'exemple représenté, le châssis 21 a une forme parallélépipédique et est disposé en avant du générateur 14, qui est lui-même disposé à droite de l'armoire des moyens de commande 16 (figure 2).

Dans une variante non représentée, le générateur 14 est monté sur le châssis 21 par l'intermédiaire de moyens d'absorption de vibrations, tels que des blocs en élastomère ou des ressorts de compression.

Comme cela est visible aux figures 1 et 2, l'automate 12 peut en outre comprendre une estrade ou plate-forme 23 surélevée qui est accessible par un opérateur pour visualiser et contrôler l'écran d'affichage des moyens de commande 16 ainsi que la cuve 12. Cette plate-forme 23 est ici située en avant des moyens de commande 16 et à gauche du châssis 21 (figures 1 et 2). Un opérateur peut accéder à cette plate-forme 21 par des marches qui sont situées sur le devant de la plate-forme.

L'automate 10 peut en outre comprendre des moyens 25 de pesée des déchets avant et/ou après leur traitement (figures 1 et 2), ces moyens de pesée 25 étant par exemple du type à bascule. Ces moyens de pesée 25 sont situés en avant du châssis 21 et à droite des marches d'accès à la plate-forme 23.

La cuve 12 de l'automate 10 est en acier et a une forme générale cylindrique d'axe longitudinal vertical. Elle a un volume de 440L dans l'exemple représenté. Ce volume est notamment fonction de la quantité de déchets à traiter et du volume maximal des emballages dans lesquels sont conditionnés les déchets lors de leur introduction dans la cuve.

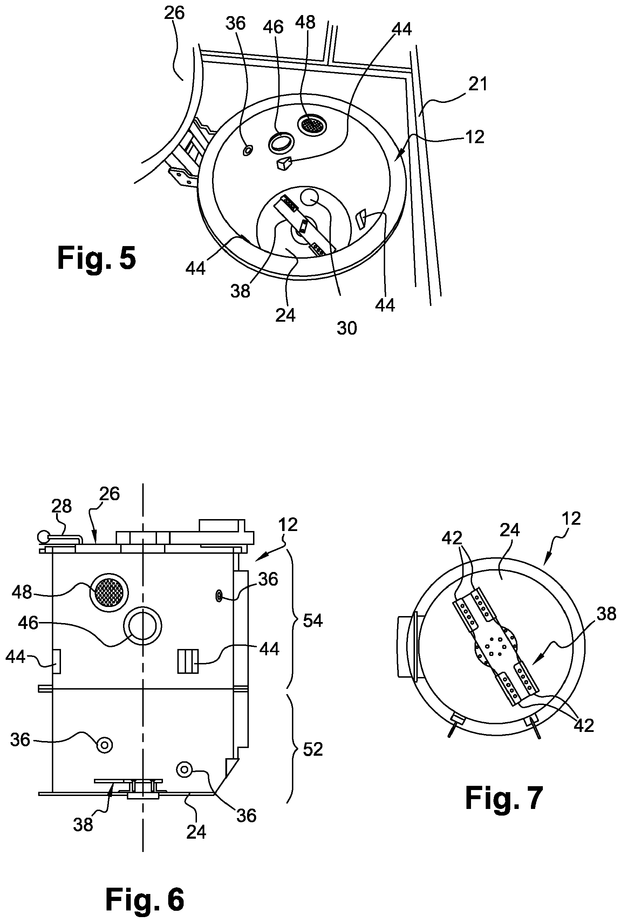

La cuve 12 est fermée à son extrémité inférieure par un fond 24 (figure 5 à 7) et à son extrémité supérieure par un couvercle 26. Ce couvercle 26 est monté pivotant à l'extrémité supérieure de la cuve entre une position de fermeture de la cuve (représentée aux figures 1 à 4 à 6) et une position d'ouverture de celle-ci (représentée en figure 5).

Le déplacement du couvercle 26 entre ces positions peut être réalisé manuellement ou par l'intermédiaire d'un moteur ou d'un vérin commandé par les moyens de commande 16 de l'automate 10. Dans le cas où l'automate 10 comprend un ou des vérins pneumatiques, ces vérins sont reliés à un système d'alimentation pneumatique qui délivre de préférence un débit d'air de l'ordre de 200L/min, à une pression de 6 bars environ.

Le couvercle 26 de la cuve 12 comprend avantageusement un hublot de contrôle à travers lequel un opérateur peut regarder pour visualiser l'état des déchets dans la cuve.

La cuve 12 est en outre équipée de moyens 28 de verrouillage de son couvercle 26. Ces moyens de verrouillage 28 empêchent l'ouverture de la cuve, en particulier lorsqu'un cycle de traitement de déchets est en cours. Ces moyens de verrouillage 28 sont de préférence actionnés par les moyens de commande 16.

Le fond 24 de la cuve 10 comprend une trappe 30 de passage et d'évacuation des déchets après traitement, vers un bac 32 de récupération et de stockage des déchets, qui est logé sous la cuve (figure 4). Le passage des déchets de la cuve 12 au bac 32 peut être réalisé simplement par gravité. Le bac 32 peut avoir une forme parallélépipédique et a par exemple une contenance de 333L et des dimensions (en mm) de 1200*800*600.

L'ouverture et la fermeture de la trappe 30 sont contrôlées par les moyens de commande 16.

Comme cela est visible en figure 4, le bac 32 est logé dans un évidement inférieur du châssis 21, cet évidement étant fermé par une porte 34 pivotante qui peut être associée à des moyens de verrouillage commandés par les moyens 16.

Des moyens de broyage des déchets sont montés au fond de la cuve 12 (figures 5 à 7) et comprennent une lame 38 montée rotative autour d'un axe vertical aligné sur l'axe de la cuve et entraînée en rotation autour de cet axe par un moteur 40 porté par le châssis (figure 3). Le moteur est situé sous la cuve 12 et est contrôlé par les moyens de commande 16. Il est de préférence conçu pour entraîner la lame 38 en rotation dans le sens horaire et dans le sens antihoraire. Le broyage des déchets peut alors s'effectuer dans les deux sens de rotation de la lame. Lorsque la lame rencontre une force de résistance trop importante en tournant dans un sens, les moyens de commande 16 peuvent imposer un sens contraire de rotation à la lame 38. Pour cela, l'arbre du moteur 40 est équipé d'un capteur de couple qui transmet un signal aux moyens de commande 16 lorsque le couple du moteur 40 dépasse un certain seuil.

La lame 38 est plane et a une forme allongée. Elle comprend sur ses bords latéraux de plus grande dimension des couteaux 42 ou éléments coupants fixés de manière amovible sur la lame, ces couteaux 42 étant par exemple au nombre de quatre. Les couteaux 42 sont facilement démontables pour pouvoir les remplacer en cas d'usure pendant une opération de maintenance. La lame 38 et/ou les couteaux 42 sont réalisés en acier et par exemple en Hardox.

La rotation de la lame, par exemple jusqu'à 2000tr/min, permet de déchiqueter tous les types de déchets (mous, durs, plastiques, tissus, métal, etc.) et de monter en température. Le temps de broyage et la vitesse de la lame permettront d'optimiser la granulométrie des déchets, c'est-à-dire la dimension des déchets/granulats après broyage (dont la taille moyenne ou le diamètre moyen est inférieur ou égal à 30mm environ).

L'automate 10 comprend de préférence des capteurs renseignant les moyens de commande 16 sur l'état, la présence et/ou la position de chaque élément déplaçable ou amovible de l'automate. L'automate 10 comprend par exemple au moins un capteur de position du couvercle 26 de la cuve 12, au moins un capteur de détection du verrouillage de la cuve 12, au moins un capteur de présence du bac de récupération 32 sous la cuve 12, et/ou au moins un capteur de position de la porte 34 d'accès au bac 32.

L'automate 10 comprend en outre des capteurs de température et/ou d'humidité qui sont logés dans la cuve 12. Dans l'exemple représenté, ces capteurs 36 sont montés sur la paroi cylindrique interne de la cuve et reliés par des moyens appropriés aux moyens de commande 16. Au moins un des capteurs de température de la cuve peut être du type infrarouge. Ce type de capteur transmet des signaux qui peuvent varier en fonction du taux d'humidité dans la cuve. Ces capteurs peuvent donc être utilisés pour déterminer indirectement le taux d'humidité dans la cuve, en comparant leur signal à celui d'un capteur classique, et donc pour détecter la présence de vapeur d'eau dans la cuve.

Dans l'exemple représenté, la paroi cylindrique de la cuve 12 comprend en outre des organes 44 en saillie, au moins une fenêtre ou un hublot 46 d'émission des micro-ondes, et au moins une grille 48 d'évacuation des effluents gazeux de la cuve.

Les organes 44 en saillie sur la paroi cylindrique de la cuve forment des obstacles pour les déchets lors de leur traitement. Lors de la rotation de la lame 38, les déchets ont tendance à être entraînés en rotation par la lame, à être centrifugés et à rester en partie haute de la cuve. Les organes 44 permettent d'éviter ou de limiter ce phénomène. Les déchets viennent au contact des organes 44 qui modifient leur trajectoire et les obligent à s'écarter de la lame et de la paroi interne de la cuve et donc à rompre les mouvements circulaires des déchets qui ont alors tendance à retomber en partie basse de la cuve pour être broyés par la lame, ce qui augmente l'efficacité du broyage.

Comme cela est visible en figure 5, chaque organe 44 est formé d'un bloc de matière qui comprend une face plaquée contre la paroi interne de la cuve et qui a en section, dans un plan perpendiculaire à l'axe longitudinal de la cuve, une forme sensiblement triangulaire dont le sommet situé radialement à l'intérieur de la cuve est arrondi.

La grille 48 permet d'évacuer les effluents gazeux de la cuve 12 pendant ou après le traitement des déchets. Cette grille 48 est reliée à une extrémité d'un conduit de gaz dont l'autre extrémité est reliée à un système 50 d'aspiration équipé d'un filtre, par exemple du type à charbon actif, destiné à retenir les molécules toxiques, nocives ou désagréables pour l'homme (figure 1). Ce conduit est avantageusement équipé d'une vanne.

Le hublot 46 de la cuve 12 à travers lequel les micro-ondes sont destinées à être émises est relié à une extrémité du guide d'onde 20 précité. Ce hublot 46 est réalisé dans un matériau approprié transparent aux micro-ondes générées par le magnétron, qui est par exemple un matériau polymère diélectrique tel que du Téflon®, PP, PEEK, etc. Le hublot 46 assure une protection mécanique du guide d'onde 20 lors de la phase de broyage.

Dans l'exemple représenté en figure 6, les capteurs 36, les organes 44, la grille 48 et le hublot 46 ont des positions particulières dans la cuve 12. La cuve 12 peut être considérée comme comprenant une demie partie inférieure 52 dans laquelle est située la lame rotative 38, et une demie partie supérieure 54. Les capteurs 36 sont situés dans les demies parties inférieure et supérieure de la cuve alors que les organes 44, la grille 48 et le hublot 46 sont situés dans la demie partie supérieure 54 de la cuve. La cuve 12 est destinée à être remplie au moins aux deux tiers de sa hauteur avec des déchets. Le niveau supérieur des déchets dans la cuve est donc situé sensiblement au dessus des organes 44 et au niveau du hublot 46 d'émission des micro-ondes.

La figure 8 représente un mode de réalisation du guide d'onde 20 de liaison du magnétron 14 au hublot 46 de la cuve 12, ce guide d'onde étant ici tubulaire et ayant en section une forme sensiblement rectangulaire. Dans l'exemple représenté, ce guide d'onde 20 comprend essentiellement quatre parties : une première portion 60 de guide d'onde, un isolateur 62 formé d'un circulateur 64 équipé d'une charge absorbante 66, une seconde portion 68 de guide d'onde et un adaptateur d'impédance 70.

La première portion 60 de guide d'onde comprend une extrémité reliée à la sortie du magnétron 14 et une extrémité opposée reliée à une des voies du circulateur 64. La seconde portion 68 de guide d'onde comprend une extrémité reliée à une autre voie du circulateur 64 et une extrémité opposée reliée à une extrémité de l'adaptateur 70 dont l'autre extrémité est reliée à la fenêtre 48 de la cuve 12.

Le circulateur 64 est du type à trois voies et comprend une première voie 70 (« 1 » en figure 9) reliée par la portion 60 de guide d'onde au magnétron 14, une deuxième voie 72 (« 2 ») reliée par la portion 68 de guide d'onde et par l'adaptateur d'impédance 70 au hublot 46 de la cuve, et une troisième voie 74 (« 3 ») reliée à la charge absorbante 66, qui est ici une charge à eau.

Les voies 70, 72 et 74 du circulateur 64 peuvent être régulièrement espacées les unes des autres et s'étendre par exemple à 120° les unes des autres autour d'un axe.

Pour former un isolateur, le circulateur comprend en son milieu, au niveau de l'axe précité, un noyau de ferrite 76 à aimentation permanente (figure 9). Ce noyau de ferrite génère un champ magnétique qui va induire un certain mouvement des ondes électromagnétiques. Les ondes incidentes générées par le magnétron 14 dans la voie 70 (« 1 ») contournent le noyau de ferrite 76 dans le sens antihoraire et sont transmises dans la voie 72 (« 2 ») pour alimenter la cuve (flèches 78). En fonctionnement, les déchets en cours de traitement, en particulier ceux en matériau métallique, peuvent réfléchir les ondes électromagnétiques émises dans la cuve. Une partie de ces ondes réfléchies sont transmises au guide d'onde 20 à travers le hublot 46 et parviennent dans la voie 72 (« 2 ») du circulateur. Ces ondes contournent alors le noyau de ferrite 76 dans le sens antihoraire et sont transmises dans la voie 74 (« 3 ») pour que leur énergie soit absorbée par la charge à eau (flèches 80). La charge à eau 66 comprend une entrée et une sortie d'eau reliées à un circuit de distribution d'eau.

L'isolateur peut être le modèle VHU207 commercialisé par la société Valvo. Cet isolateur a les paramètres de fonctionnement suivant : fréquence de 2425-2475MHz, isolation de 23dB min et puissance de 3kW. Il peut être relié au refroidisseur 22 à eau précité en vue de son refroidissement.

La figure 10 représente un mode de réalisation de l'adaptateur d'impédance 70 du guide d'onde 20. Cet adaptateur d'impédance 70 est ici du type à éléments (vis, piston, tige, *stubs*, etc.) plongeants, chaque élément 82 traversant une paroi d'une portion de guide d'onde et étant engagé ou enfoncé dans ce guide d'onde de manière contrôlée de façon à ce que la partie enfoncée dans la portion de guide d'onde forme un obstacle aux ondes électromagnétiques passant à travers cette portion et provoque ainsi des réflexions partielles des ondes.

L'adaptateur d'impédance 70 permet d'optimiser les paramètres de fonctionnement du magnétron pour limiter notamment la réflexion des ondes électromagnétiques par les déchets et la perte d'énergie des ondes réfléchies et absorbées par la charge, par le réglage optimal de la profondeur de pénétration des éléments plongeants précités dans la portion de guide d'onde. L'association de plusieurs de ces éléments réfléchissants et de leur enfoncement dans la portion de guide d'onde permet donc d'adapter les impédances, vues par le magnétron, des charges constitutives du circuit complet (afin d'obtenir un rendement maximum d'absorption de l'énergie par les déchets dans la cuve).

Les différents éléments montés sur la paroi cylindrique interne de la cuve 12 peuvent perturber la propagation des ondes électromagnétiques, ce qui peut engendrer des fuites d'onde. Pour limiter le taux de fuite à moins de 5mW/cm², les fuites d'onde sont piégées par des moyens tels que des pièges quart d'onde, des pièges comportant des matériaux absorbants et/ou des joints formés de tresses métalliques. Les pièges quart d'onde sont par exemple réalisés en concevant la cuve avec des jeux optimums de quelques millimètres entre ses composants, et par exemple entre la cuve et le couvercle 26. Un joint d'étanchéité de blindage électromagnétique, en tresses métalliques, peut en outre être monté sur le bord périphérique du couvercle et est destiné à être serré entre ce bord et le bord périphérique supérieur de la cuve en position de fermeture de celle-ci. La trappe 30 d'évacuation des déchets au fond de la cuve 12 peut comprendre une cheminée cylindrique dont le diamètre et la longueur sont déterminés, selon la fréquence utilisée, pour piéger les fuites d'onde.

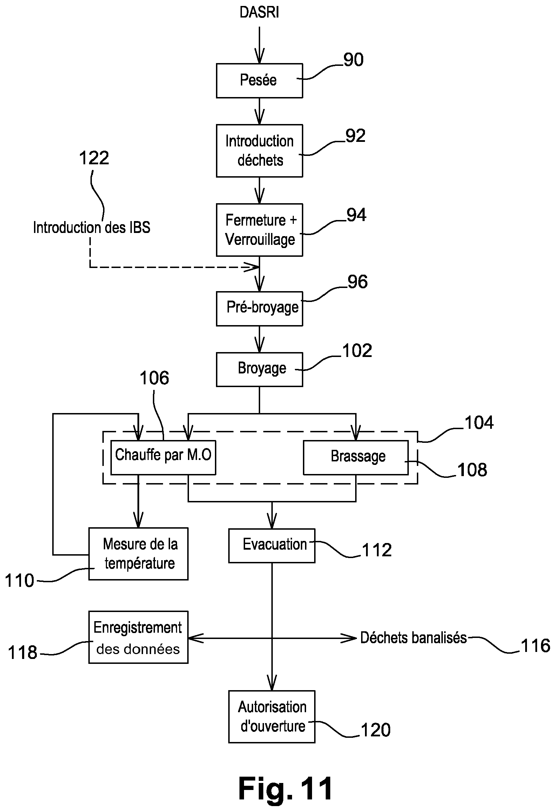

On se réfère maintenant à la figure 11 qui est un organigramme de présentation des étapes du procédé selon l'invention de traitement des déchets du type DASRI.

Une première étape du procédé selon l'invention consiste à peser les déchets du type DASRI par l'intermédiaire des moyens de pesée 25 précités (étape 90). Ces déchets sont ensuite introduits dans la cuve 12 (étape 92), dont le couvercle est préalablement ouvert par les moyens de commande 16 ou par un opérateur. L'introduction ou le chargement des déchets dans la cuve 12 peut être réalisé manuellement ou au contraire de façon automatique à l'aide d'un système robotisé par exemple. Le couvercle de la cuve est ensuite fermé manuellement ou par les moyens de commande 16, ces derniers contrôlant ensuite le verrouillage du couvercle (étape 94). Les moyens de commande 16 contrôlent également la présence du bac 32 de récupération des déchets après traitement, sous la cuve 12, et la fermeture et le verrouillage de la porte 34 d'accès à ce bac.

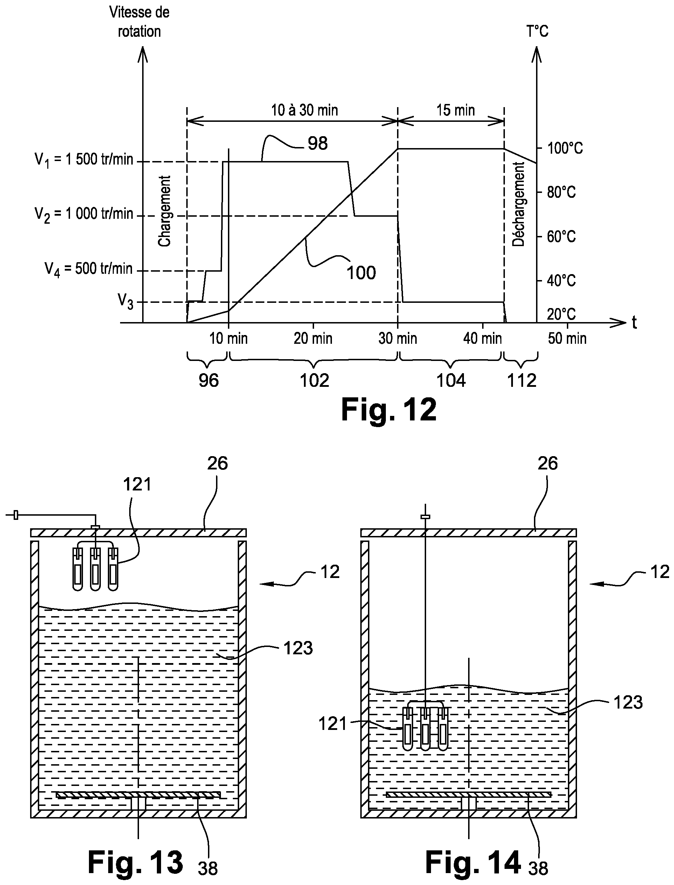

L'étape 96 suivante consiste à pré-broyer les déchets par rotation de la lame 38 en vue de réduire leur granulométrie. Comme cela est visible en figure 12, l'étape de pré-broyage a une durée inférieure ou égale à 10 minutes. Le pré-broyage des déchets est effectué pendant 4 à 5min par rotation de la lame 38 à une vitesse V3 puis pendant 4 à 5min par rotation de la lame 38 à une vitesse V4 qui est supérieure à V3. V3 est par exemple de 250tr/min et V4 est par exemple de 500tr/min (voir les deux premiers paliers de la courbe 98 en figure 12). Le pré-broyage des déchets provoque une montée en température des déchets à l'intérieur de la cuve 12, jusqu'à environ 30°C (courbe 100 en figure 12).

Les déchets sont ensuite broyés (étape 102) pendant 10 à 30 minutes environ, et par exemple pendant 20min. Le broyage est effectué pendant 15min environ par rotation de la lame 38 à une vitesse V1 supérieure à V3 et V4, puis pendant 5min environ par rotation de la lame à une vitesse V2 qui est inférieure à V1 et supérieure à V3 et V4. V1 est par exemple de 1500tr/min et V2 est par exemple de 1000tr/min (voir les deux paliers de la courbe 98 en figure 12, à 1500 et 1000tr/min, respectivement). Le broyage des déchets provoque une montée en température des déchets à l'intérieur de la cuve 12, jusqu'à 90-95°C environ (courbe 100 en figure 12). Cette montée en température lors des étapes de pré-broyage et de broyage est notamment due à la puissance dissipée par le moteur d'entraînement de la lame rotative et par le choc de la lame 38 sur les déchets.

L'étape 104 suivante consiste à désinfecter les déchets par la combinaison du chauffage des déchets (étape 106) par les micro-ondes émises dans la cuve 12 et par le brassage des déchets (étape 108).

Les micro-ondes sont émises dans la cuve 12 pour chauffer les déchets et maintenir une température prédéterminée dans la cuve, qui est de 95°C environ dans l'exemple représenté. Les capteurs de la cuve 12 renseignent les moyens de commande 16 sur la température des déchets (étape 110). Les moyens de commande 16 peuvent ajuster les paramètres de fonctionnement du générateur 14 en fonction des signaux transmis par ces capteurs.

Le brassage des déchets est réalisé pendant 15min environ par rotation de la lame 38 à la vitesse V3 ou à une vitesse légèrement inférieure à V3 (par exemple de l'ordre de 200 à 250tr/min). Ce brassage permet d'uniformiser l'exposition des déchets aux micro-ondes et de garantir une température uniforme dans la cuve.

Lorsque la désinfection des déchets est terminée, les moyens de commande 16 arrêtent le générateur 14 ainsi que le broyage et le brassage des déchets.

Les moyens de commande 16 contrôlent alors l'ouverture de la vanne de liaison de la grille 48 de la cuve au système de filtration 50, de façon à ce que les gaz, et en particulier la vapeur d'eau, contenus dans la cuve traversent la grille et soient filtrés par le système 50. Pour vaporiser et éliminer au maximum l'eau contenue dans les déchets, on peut effectuer un cycle de quelques minutes de rotation de la lame à grande vitesse (par exemple V1 ou V2) avec la vanne précitée ouverte. On peut obtenir de cette manière une réduction significative de la masse des déchets, par exemple de l'ordre de 20%. Comme expliqué dans ce qui précède, un capteur infrarouge peut renseigner les moyens de commande 16 sur le taux d'humidité dans la cuve. Dans le cas où ce taux est relativement important, l'étape de vaporisation et de rotation de la lame peut être maintenue jusqu'à ce que le taux soit situé en dessous d'un certain seuil. Si les déchets sont trop secs (taux d'humidité faible et température supérieure à un certain seuil), les moyens de commande 16 peuvent actionner des buses d'alimentation en eau de la cuve, pour arroser et humidifier les déchets.

Les déchets peuvent ensuite être évacués de la cuve par ouverture de la trappe d'évacuation 30 précitée (étape 112). Les moyens 16 commandent l'ouverture de la trappe 30 ainsi que la rotation de la lame 38 à une vitesse faible pour entraîner les déchets à travers la trappe ouverte. Les déchets appelés déchets « banalisés » sont alors évacués ou déchargés vers le bac de récupération 32 situé sous la cuve 12 (étape 116). Pour avoir accès au bac 32, un opérateur doit d'abord requérir le déverrouillage et l'ouverture de la porte 34 auprès des moyens de commande 16.

Le couvercle 26 de la cuve 12 est ensuite déverrouillé (étape 120) et déplacé en position ouverte manuellement ou par les moyens de commande 16.

La durée totale d'un cycle de traitement de déchets est en général de l'ordre de 35 à 50min et dépend notamment de la température initiale dans la cuve 12, avant le début du traitement.

Les moyens 16 qui commandent l'ensemble des équipements de l'automate 10 et qui contrôlent le déroulement du procédé de traitement peuvent alors enregistrer et sauvegarder les données propres aux déchets traités, telles que les paramètres de désinfection, le poids des déchets, leur provenance, etc. (étape 118). Il n'est pas nécessaire qu' un opérateur intervienne pendant le déroulement du procédé, qui est entièrement automatisé, ce qui est très avantageux. L'opérateur peut suivre le déroulement du procédé de traitement par le biais de l'écran de contrôle 18 des moyens de commande 16. L'opérateur peut, via cet écran 18, lancer ou arrêter un cycle de traitement, et avoir accès aux paramètres du procédé, aux résultats du traitement, etc.

Les figures 13 et 14 représentent une étape facultative et/ou périodique du procédé selon l'invention, cette étape consistant à vérifier la capacité de désinfection du procédé et de l'automate.

Ce test de désinfection comprend au moins deux sous-étapes dont la première est réalisée avant l'étape 96 précitée de pré-broyage des déchets. La première sous-étape 122 consiste à introduire dans la cuve 12 des échantillons témoins de stérilisation (appelés IBS pour Indicateurs Bactériologiques de Stérilisation), qui sont placés dans des tubes à essai 121 et qui comprennent des germes ou des bactéries destinés à être détruits par le procédé de traitement. Avant l'étape de pré-broyage, les échantillons doivent être maintenus en position haute dans la cuve de façon à ne pas être au contact des déchets 123 (figure 13). En pratique, les tubes peuvent être suspendus dans cette position au moyen d'un fil en polypropylène. Lorsque le broyage est terminé, c'est-à-dire après l'étape 102, les échantillons sont plongés dans les déchets 123 situés au fond de la cuve (figure 14) pour être soumis aux micro-ondes en même temps que les déchets.

Après l'évacuation des déchets de la cuve et le déverrouillage et l'ouverture de son couvercle, un opérateur peut récupérer les échantillons en vue de leur analyse. Cette analyse permettra de déterminer si le procédé de traitement est suffisant pour désinfecter les déchets et valider le fonctionnement de l'automate pour une période donnée, par exemple de 3 mois. Un test de désinfection peut être réalisé tous les trois mois.

Dans un cas particulier de réalisation de l'invention, l'ensemble des composants de l'automate 10 occupe un volume parallélépipédique ayant les dimensions suivantes : longueur 3m, largeur 1,7m et hauteur 2m. Il a un poids de 1600kg, est alimenté par un réseau 400V (triphasé), et a une consommation moyenne de 10kW. La cuve peut contenir de 360 à 440L de déchets ce qui représente entre 32 et 35kg de déchets, et permet de réduire la population bactérienne et virale des déchets d'un facteur 5 log10. Ce type d'automate est particulièrement adapté pour être installé dans un centre de soins comportant entre 100 et 400 lits et générant environ 250kg/jour de déchets à banaliser.

## Revendications

1. Automate (10) de traitement de déchets, en particulier de banalisation de déchets d'activités de soins à risques infectieux, comprenant une cuve (12) montée sur un châssis (21) et destinée à être alimentée en déchets, des moyens (38) de broyage des déchets montés au fond de la cuve, des moyens de chauffage des déchets en vue de leur désinfection, et des moyens informatisés (16) de commande des moyens de broyage et de chauffage, les moyens de chauffage comprenant au moins un générateur (14) de micro-ondes dont la sortie est reliée à une extrémité d'un guide d'onde (20) dont l'autre extrémité débouche dans la cuve, le générateur étant porté par des moyens de support indépendants du châssis ou montés sur le châssis par des moyens absorbant les vibrations de la cuve lors du broyage des déchets.

2. Automate selon la revendication 1, **caractérisé en ce que** le guide d'onde (20) débouche à son extrémité opposée au générateur sur un hublot (46) de la cuve (12), à travers lequel sont émises les micro-ondes.

3. Automate selon la revendication 2, **caractérisé en ce que** la cuve (12) comprend une paroi sensiblement cylindrique dont l'axe de révolution s'étend sensiblement verticalement, et **en ce que** le hublot (46) est situé dans une demie partie supérieure (54) de la cuve.

4. Automate selon l'une des revendications précédentes, **caractérisé en ce que** le générateur (14) est un magnétron qui fonctionne à une fréquence comprise entre 1 et 3 GHz, et par exemple de 2,45 GHz environ.

5. Automate selon l'une des revendications précédentes, **caractérisé en ce que** le guide d'onde (20) comprend un isolateur (62) destiné à autoriser le passage des ondes (78) émises par le générateur (14) vers la cuve (12) et à empêcher le retour des ondes réfléchies (80) depuis la cuve vers le générateur.

6. Automate selon la revendication 5, **caractérisé en ce que** le guide d'onde (20) comprend un circulateur (64) à trois voies équipé d'un isolateur (62) à charge absorbante (66), le circulateur comportant une première voie (70) reliée par une portion (60) de guide d'onde à la sortie du générateur (14), une seconde voie (72) reliée par une portion (68) de guide d'onde à la cuve (12), et une troisième voie (74) reliée à la charge absorbante, l'isolateur comportant un noyau en ferrite (76) à aimentation permanente disposé au centre du circulateur et destiné à forcer les ondes incidentes provenant de la première voie à passer dans la seconde voie et les ondes réfléchies de la seconde voie à passer dans la troisième voie.

7. Automate selon l'une des revendications précédentes, **caractérisé en ce que** le guide d'onde (20) comprend un adaptateur d'impédance (70), par exemple du type à piston(s) ou tige(s) (82) plongeant(s).

8. Automate selon l'une des revendications précédentes, **caractérisé en ce que** la cuve (12) comprend des moyens empêchant ou limitant les fuites d'ondes tels que des pièges quart d'onde, des pièges comportant des matériaux absorbants, des joints formés de tresses métalliques, et /ou des jeux résiduels de montage entre certaines pièces de la cuve afin d'éviter la formation d'arcs électriques.

9. Automate selon l'une des revendications précédentes, **caractérisé en ce qu'**il comprend un ou plusieurs des équipements suivants :
- au moins un capteur (36) de la température et/ou de l'humidité à l'intérieur de la cuve (12) ;
- un système (22) de refroidissement du générateur par circulation d'eau autour, à proximité ou à l'intérieur de celui-ci ;
- un ou plusieurs systèmes (50) de filtration de gaz permettant de traiter les vapeurs et les effluents olfactifs générés lors du traitement des déchets, ce système comportant une entrée reliée par un conduit à un orifice (48) d'évacuation de gaz de la cuve (12), dont l'ouverture peut être commandée par une vanne ;
- des moyens (25) de pesée des déchets avant leur introduction dans la cuve (12) et/ou après leur traitement ;
- une plate-forme (23) surélevée de contrôle qui est accessible par un opérateur, et qui est située à côté du châssis (21) de support de la cuve (12) et des moyens de commande (16) ;
- des moyens (32) de récupération et de stockage des déchets après traitement, ces moyens de récupération étant logés sous la cuve (12) qui comporte au niveau ou au voisinage de son fond au moins une trappe (30) d'évacuation des déchets et de vidange de la cuve ;
- au moins un organe (44) en saillie sur une paroi cylindrique interne de la cuve (12), cet organe formant un obstacle pour les déchets lors du broyage ; et/ou
- un couvercle (26) de fermeture étanche de la cuve (12), ce couvercle étant monté pivotant à l'extrémité supérieure de la cuve entre une position d'ouverture et une position de fermeture de la cuve, et des moyens (28) de verrouillage du couvercle en position de fermeture, qui sont commandés par les moyens de commande (16).

10. Procédé automatisé de traitement de déchets au moyen d'un automate (10) selon l'une des revendications 1 à 9, cet automate comportant des moyens (38) de broyage des déchets du type à lame tournante, **caractérisé en ce qu'**il comprend les étapes consistant à :
a) introduire les déchets dans la cuve (12) ;
b) broyer les déchets par rotation de la lame à une vitesse V1 donnée, le broyage produisant de la chaleur chauffant les déchets ;
c) émettre des micro-ondes dans la cuve en brassant les déchets par rotation de la lame à une vitesse V3 inférieure à V1, pour chauffer les déchets et les maintenir à une température de désinfection pendant une durée déterminée ; et
d) évacuer les déchets de la cuve.

11. Procédé selon la revendication 10, **caractérisé en ce qu'**il comprend une ou plusieurs des étapes suivantes :
- avant l'étape a) et/ou après l'étape d), une étape de pesée des déchets ;
- après l'étape a), une étape de fermeture et de verrouillage du couvercle (26) de la cuve (12) ;
- avant l'étape b), une étape de pré-broyage des déchets par rotation de la lame (38) à une vitesse V4 inférieure à V1 ;
- pendant l'étape c), une étape de mesure de la température à l'intérieur de la cuve (12) ;
- après l'étape d), une étape d'enregistrement des données du traitement par les moyens de commande (16) ; et/ou
- une étape de test de désinfection comportant, avant l'étape c), une sous-étape d'introduction d'échantillons témoins de désinfection dans la cuve (12), et, après l'étape d), une sous-étape de récupération des échantillons précités en vue de leur analyse.

## Patentansprüche

1. Abfallbehandlungsautomat (10), insbesondere der Banalisierung von Abfällen von Pflegeaktivitäten mit Infektionsrisiko, umfassend einen Bottich (12), auf einem Gestell (21) montiert und dazu bestimmt, mit Abfällen gespeist zu werden, am Boden des Bottichs montierte Abfallzerkleinerungsmittel (38), Abfallerwärmungsmittel zwecks deren Desinfizierung und computerisierte Steuerungsmittel (16) der Zerkleinerungs- und Erwärmungsmittel, wobei die Erwärmungsmittel mindestens einen Mikrowellengenerator (14) umfassen, von dem der Auslass mit einem Ende einer Wellenführung (20) verbunden ist, von dem das andere Ende in dem Bottich mündet, wobei der Generator unabhängig von dem Gestell durch Trägermittel getragen oder durch die Schwingungen des Bottichs bei der Zerkleinerung der Abfälle absorbierende Mittel auf dem Gestell montiert wird.

2. Automat nach Anspruch 1, **dadurch gekennzeichnet, dass** die Wellenführung (20) an ihrem gegenüberliegenden Ende am Generator an einer Sichtscheibe (46) des Bottichs (12) mündet, durch welche die Mikrowellen hindurch emittiert werden.

3. Automat nach Anspruch 2, **dadurch gekennzeichnet, dass** der Bottich (12) eine im Wesentlichen zylindrische Wand umfasst, von der die Drehachse sich im Wesentlichen vertikal erstreckt, und dadurch, dass die Sichtscheibe (46) sich in einem oberen Halbteil (54) des Bottichs befindet.

4. Automat nach einem der vorstehenden Ansprüche, **dadurch gekennzeichnet, dass** der Generator (14) ein Magnetron ist, der bei einer Frequenz im Bereich zwischen 1 und 3 GHz, und zum Beispiel von ungefähr 2,45 GHz funktioniert.

5. Automat nach einem der vorstehenden Ansprüche, **dadurch gekennzeichnet, dass** die Wellenführung (20) einen Isolator (62) umfasst, der dazu bestimmt ist, den Durchgang der Wellen (78), die durch den Generator (14) in Richtung des Bottichs (12) emittiert werden, zu ermöglichen, und die Rückkehr der von dem Bottich in Richtung des Generators reflektierten Wellen (80) zu verhindern.

6. Automat nach Anspruch 5, **dadurch gekennzeichnet, dass** die Wellenführung (20) einen Drei-Wege-Zirkulator (64), ausgestattet mit einem Isolator (62) mit absorbierendem Füllstoff (66), umfasst, wobei der Zirkulator einen durch einen Abschnitt (60) der Wellenführung am Auslass des Generators (14) verbundenen ersten Weg (70), einen zweiten, durch einen Abschnitt (68) der Wellenführung mit dem Bottich (12) verbundenen Weg (72) und einen dritten, mit der Absorptionsladung verbundenen dritten Weg (74) beinhaltet, wobei der Isolator einen Ferritkern (76) zur permanenten Magnetisierung beinhaltet, angeordnet in der Mitte des Zirkulators und dazu bestimmt, die von dem ersten Weg stammenden auftreffenden Wellen zu zwingen, in den zweiten Weg über zu gehen, und die von dem zweiten Weg reflektierten Wellen, in den dritten Weg über zu gehen.

7. Automat nach einem der vorstehenden Ansprüche, **dadurch gekennzeichnet, dass** die Wellenführung (20) einen Impedanzadapter (70) umfasst, zum Beispiel vom Kolben- oder Eintauchstab-/Eintauchstäbetyp (82).

8. Automat nach einem der vorstehenden Ansprüche, **dadurch gekennzeichnet, dass** der Bottich (12) Mittel umfasst, die das Entweichen von Wellen verhindern oder beschränken, wie Viertelwellenfallen, Fallen, die absorbierende Materialien beinhalten, aus metallischen Geflechten gebildete Verbindungen und/oder verbleibende Spielräume von der Montage zwischen bestimmten Stücken des Bottichs, um die Bildung von Lichtbögen zu vermeiden.

9. Automat nach einem der vorstehenden Ansprüche, **dadurch gekennzeichnet, dass** er eine oder mehrere der folgenden Ausstattungen umfasst:
- mindestens einen Temperatur- und/oder Feuchtigkeitssensor (36) im Inneren des Bottichs (12);
- ein Kühlungssystem (22) des Generators, durch Zirkulation von Wasser um, in der Nähe oder im Inneren desselben;
- ein oder mehrere Gasfiltrationssysteme (50), die gestatten, die Dämpfe und die olfaktorischen Ausströmungen, die bei der Behandlung der Abfälle erzeugt werden, zu behandeln, wobei dieses System einen durch eine Leitung mit einer Austrittsöffnung (48) zur Gasevakuierung des Bottichs (12) verbundenen Einlass beinhaltet, von dem die Öffnung durch eine Absperrklappe gesteuert werden kann; - Mittel (25) des Wiegens der Abfälle vor deren Einführung in den Bottich (12) und/oder nach deren Behandlung;
- eine erhöhte Steuerungsplattform (23), die durch einen Bediener zugänglich ist und die sich an der Seite des Trägergestells (21) des Bottichs (12) und der Steuerungsmittel (16) befindet;
- Wiedergewinnungs- und Lagerungsmittel (32) der Abfälle nach der Behandlung, wobei diese Wiedergewinnungsmittel unter dem Bottich (12) beherbergt sind, der auf Höhe oder benachbart zu seinem Boden mindestens eine Evakuierungsklappe (30) der Abfälle und der Entleerung des Bottichs aufweist; - mindestens ein auf einer internen zylindrischen Wand des Bottichs (12) hervorstehendes Bauteil (44), wobei dieses Bauteil für die Abfälle bei der Zerkleinerung ein Hindernis bildet; und/oder
- einen dichten Verschlussdeckel (26) des Bottichs (12), wobei dieser Deckel schwenkbar am oberen Ende des Bottichs zwischen einer Öffnungsposition und einer Verschlussposition des Bottichs montiert ist, und Verriegelungsmittel (28) des Deckels in Verschlussposition, die durch die Steuerungsmittel (16) gesteuert werden.

10. Automatisiertes Verfahren der Abfallbehandlung mittels eines Automaten (10) nach einem der Ansprüche 1 bis 9, wobei dieser Automat Abfallzerkleinerungsmittel (38) des Drehklingentyps beinhaltet, **dadurch gekennzeichnet, dass** die Schritte aus Folgendem bestehen:
a) Einführen der Abfälle in den Bottich (12);
b) Zerkleinern der Abfälle durch Drehung der Klingen bei einer gegebenen Geschwindigkeit V1, wobei die Zerkleinerung Wärme erzeugt, die die Abfälle erwärmt;
c) Emittieren von Mikrowellen in den Bottich beim Umwälzen der Abfälle durch Drehung der Klingen bei eine Geschwindigkeit V3, geringer als V1, um die Abfälle zu erwärmen und sie während eine bestimmten Dauer bei einer Desinfektionstemperatur zu halten; und
d) Abführen der Abfälle aus dem Bottichs.

11. Verfahren nach Anspruch 10, **dadurch gekennzeichnet, dass** es einen oder mehrere der folgenden Schritte umfasst:
- vor dem Schritt a) und/oder nach dem Schritt d), einen Schritt des Wiegens der Abfälle;
- nach dem Schritt a), einen Schritt des Schließens und des Verriegelns des Deckels (26) des Bottichs (12);
- vor dem Schritt b), einen Schritt der Vorzerkleinerung der Abfälle durch Drehung der Klingen (38) bei einer Geschwindigkeit V4, geringer als V1;
- während Schritt c), einen Schritt des Messens der Temperatur im Inneren des Bottichs (12);
- nach Schritt d), einen Schritt des Registrierens der Daten der Behandlung durch die Steuerungsmittel (16); und/oder
- einen Schritt des Prüfens der Desinfektion, beinhaltend, vor Schritt c), einen Teilschritt des Einführens von Kontrollproben der Desinfektion in den Bottich (12), und, nach dem Schritt d), einen Teilschritt der Wiedergewinnung der vorgenannten Proben zwecks deren Analyse.

## Claims

1. Automaton (10) for treating waste, in particular for banalizing waste from healthcare activities with risks of infection, comprising a tank (12) mounted on a chassis (21) and intended to be provided with waste, means (38) for crushing waste mounted at the bottom of the tank, means for heating waste in view of the disinfection thereof, and computerised means (16) for controlling crushing and heating means, the heating means comprising at least one microwave generator (14) of which the output is connected to an end of a waveguide (20) of which the other end opens into the tank, the generator being carried by independent means regarding the chassis or mounted on the chassis by means absorbing vibrations of the tank during the crushing of waste.

2. Automaton according to claim 1, **characterised in that** the waveguide (20) opens at the end thereof opposite the generator on a window (46) of the tank (12), through which microwaves are emitted.

3. Automaton according to claim 2, **characterised in that** the tank (12) comprises a substantially cylindrical wall of which the axis of revolution extends substantially vertically, and **in that** the window (46) is located in an upper half-portion (54) of the tank.

4. Automaton according to one of the preceding claims, **characterised in that** the generator (14) is a magnetron which functions at a frequency of between 1 and 3GHz, and for example, around 2.45GHz.

5. Automaton according to one of the preceding claims, **characterised in that** the waveguide (20) comprises an insulator (62) intended to enable the passage of waves (78) emitted by the generator (14) to the tank (12) and to prevent the return of reflected waves (80) from the tank to the generator.

6. Automaton according to claim 5, **characterised in that** the waveguide (20) comprises a three-path circulator (64) equipped with an absorbent load (66) insulator (62), the circulator comprising a first path (70) connected by a waveguide portion (60) to the output of the generator (14), a second path (72) connected by a waveguide portion (68) to the tank (12), and a third path (74) connected to the absorbent load, the insulator comprising a permanently magnetised ferrite core (76) arranged at the centre of the circulator and intended to force incident waves coming from the first path to pass into the second path and the waves reflected from the second path to pass into the third path.

7. Automaton according to one of the preceding claims, **characterised in that** the waveguide (20) comprises an impedance adaptor (70), for example of the piston or immersion tube type (82).

8. Automaton according to one of the preceding claims, **characterised in that** the tank (12) comprises means preventing or limiting the wave leakages such as quarter-wave traps, traps comprising absorbent materials, seals formed of metal inlays, and/or residual mounting clearances between certain parts of the tank in order to avoid the formation of electric arcs.

9. Automaton according to one of the preceding claims, **characterised in that** it comprises one or more of the following items of equipment:
- at least one temperature sensor (36) and/or humidity sensor inside the tank (12);
- a system (22) for cooling the generator by circulating water around, in the proximity or inside it;
- one or more systems (50) for filtering gas making it possible to treat steam and olfactory effluents generated by the treatment of waste, this system comprising an input connected by a conduit to an orifice (48) for evacuating gas from the tank (12), of which the opening can be controlled by a valve;
- means (25) for weighing waste before it is introduced into the tank (12) and/or after the treatment thereof;
- a raised control platform (23) which is accessible by an operator, and which is located on the side of the chassis (21) for supporting the tank (12) and control means (16);
- means (32) for recovering and storing waste after treatment, these recovery means being housed under the tank (12) which comprises, at the level of or near the bottom thereof, at least one flap (30) for evacuating waste and emptying the tank;
- at least one member (44) protruding on an inner cylindrical wall of the tank (12), this member forming an obstacle for waste during crushing; and/or
- a sealed closing cover (26) of the tank (12), this cover being mounted pivoting at the upper end of the tank between an opening position and a closing position of the tank, and means (28) for locking the cover in the closing position, which are controlled by the control means (16).

10. Automated method for treating waste by means of an automaton (10) according to one of claims 1 to 9, this automaton comprising means (38) for crushing waste of the rotating blade type, **characterised in that** it comprises steps consisting of:
a) introducing waste into the tank (12);
b) crushing waste by rotating the blade at a given speed V1, the crushing step generating heat heating the waste ;
c) emitting microwaves into the tank by stirring waste by rotating the blade at a speed V3 less than V1, to heat the waste and to keep it at a disinfection temperature for a determined duration; and
d) evacuating waste from the tank.

11. Method according to claim 10, **characterised in that** it comprises one or more of the following steps:
- before step a) and/or after step d), a step of weighing the waste;
- after step a), a step of closing and locking the cover (26) of the tank (12);
- before step b), a step of pre-crushing the waste by rotating the blade (38) at a speed V4 less than V1;
- during step c), a step of measuring the temperature inside the tank (12);
- after step d), a step of recording data from the treatment by the control means (16); and/or
- a step of testing disinfection comprising, before step c), a sub-step of introducing disinfection control samples into the tank (12) and after step d), a sub-step of recovering the abovementioned samples in view of the analysis thereof.
